# EUROPEAN PATENT SPECIFICATION

(11) **EP 4 140 519 B1**
(45) Date of publication and mention of the grant of the patent: **07.05.2025**
(21) Application number: 22193186.8
(22) Date of filing: 31.08.2022
(51) Int. Cl.: A61M 5/142, A61M 39/12

(54) **INTRAVENOUS TUBING UPPER FITMENT, SYSTEM AND LOADING METHOD**
ANSCHLUSS EINES INTRAVENÖSEN SCHLAUCHES, SYSTEM UND LADEVERFAHREN
RACCORD SUPÉRIEUR DE TUBULURE INTRAVEINEUSE, SYSTÈME ET MÉTHODE DE CHARGEMENT

(30) Priority: 31.08.2021 US 202163239327 P
(43) Date of publication of application: 01.03.2023
(73) Proprietor: CareFusion 303, Inc., San Diego, CA 92130 (US)
(72) Inventor: ABAL, Daniel M., San Diego, 92130 (US)
(74) Representative: Epping - Hermann - Fischer

(56) References cited:
- US-A1- 2007 076 401
- US-B2- 11 045 599

## Description

### BACKGROUND

The present disclosure generally relates to an intravenous (IV) set or infusion pump flow control, and in particular an IV tubing fitment for securing an IV tube to a fluid delivery device.

US 11 045 599 B2 relates to connectors which are intended for use in the medical field such as in perfusion devices for intravenous or arterial catheters, and discloses a an intravenous, IV, tubing fitment, comprising: a fluid delivery device including a body forming, in part, a mating recess and a door, wherein the door forms part of the mating recess, and an intravenous, IV, tubing fitment, comprising a unitary body configured to couple with the mating recess of the fluid delivery device.

Document US 2007/076401 A1 discloses connectors which are intended for use in the medical field such as in perfusion devices for intravenous or arterial catheters. An entry diameter and a thread crest diameter of the connectors are selected in relation to the corresponding diameters of standard connectors such that the assembly of a male connector (A, C) or a female connector (B, D) respectively with a standard female or male connector is prevented. Medical treatments often include the infusion of a medical fluid (e.g., a saline solution or a liquid medication) to patients using an intravenous (IV) catheter that is connected though an arrangement of flexible tubing and fittings, commonly referred to as an "IV set," to a source of fluid, for example, an IV bag.

IV sets are loaded into a medical device, such as an infusion pump, that controls the flow rate through the tubing. Due to the designs of existing IV sets, the IV sets can be unintentionally misloaded causing the tubing of the IV sets to be pulled, twisted, entangled, or otherwise interfered with. IV sets that are misloaded result in portions of the tubing to be reshaped, which in turn causes a change or occludes a flow rate produced by the medical device. For example, IV set that are misloaded can be pulled causing the tubing to stretch and narrow a diameter of the tubing. Misloaded IV sets can result in inaccurate flow rates, uncontrolled flow rates, over infusion, under infusion, and/or a number of other issues.

Thus, it is desirable to provide an IV set that reduces the opportunities for misloading into a medical device.

### SUMMARY

The invention is related to an intravenous tubing fitment, a system and a method for loading an intravenous set to a fluid delivery device as set out in the appended set of claims. Various implementations of systems, methods, and devices within the scope of the appended claims each have several aspects, no single one of which is solely responsible for the attributes described herein. Without limiting the scope of the appended claims, after considering this disclosure, and particularly after considering the section entitled "Detailed Description" one will understand how the aspects of some implementations are used to prevent an IV set from being misloaded to provide accurate and consistent flow rates. One or more implementations provide an IV tubing fitment including a unitary body that is configured to be received by a pocket of a fluid delivery device in a single configuration. The IV tubing fitment is configured such that the fluid delivery device is unable to use the IV tubing fitment if misloaded. More specifically, the IV tubing fitment is designed such that a fluid delivery device cannot be forced closed if the IV tubing fitment is misloaded. By not allowing itself to be misloaded, the IV tubing fitment prevents a tubing of the IV set from being excessively pulled, stretched, entangled, kinked, etc. by securely holding the IV set in place within the fluid delivery device. Thus, the IV tubing fitment prevents the diameter, wall thickness, or length of the tubing of the IV set from being altered. The IV tubing fitment further allows the tubing of the IV set to rotate about a longitudinal axis while held in place within the fluid delivery device. This allows the tubing of the IV set to be rotate around without being fixed to a single position. As such, the IV tubing fitment is able to reduce or eliminate inaccurate or uncontrollable flow rates caused by the misloading of IV sets, as well as reduce or eliminate the possibility of over infusion or under infusion caused by the of IV sets. In one aspect, an intravenous (IV) tubing fitment is provided. The IV tubing fitment includes a unitary body. The unitary body includes a first end configured to couple to a first portion of a tubing, a substantially hemispherical surface adjacent to the first end, and a cylindrical body adjacent to the substantially hemispherical surface. The cylindrical body including a plurality of wings and is configured to receive a second portion of the tubing. The unitary body is configured to be received into a mating recess of a fluid delivery device such that the substantially hemispherical surface is uniformly mated with a substantially identical surface of the mating recess, and the cylindrical body is positioned outside of the mating recess. In some implementations, the unitary body is molded. In some implementations, the fluid delivery device is an infusion pump. In some implementations, the unitary body, when received by the mating recess, rotates about a longitudinal axis at least 270 degrees in a clockwise and counterclockwise direction. In some implementations, the unitary body rotates about the longitudinal axis a full 360 degrees in each direction. In some implementations, the substantially hemispherical surface includes an outer rim that is configured to engage with a door of the fluid delivery device. In some implementations, the outer rim, when engaged with the door of the fluid delivery device, restricts movement of the unitary body, in at least one of a longitudinal and a latitudinal directions.

In some implementations, the substantially hemispherical surface is uniformly mated with the substantially identical surface of the mating recess only when the first end of the unitary body is received within a pocket of the mating recess preventing the IV tubing fitment from being misloaded. In some implementations, when the first end of the unitary body is received within the pocket of the mating recess, the first portion of the tubing coupled with the first end of the unitary body is aligned along a center of the mating recess. In some implementations, the substantially hemispherical surface has a diameter that is substantially equal to a diameter of the mating recess, the diameter of the substantially hemispherical surface preventing the fluid delivery device from closing when the substantially hemispherical surface is not uniformly mated with the substantially identical surface of the mating recess.

In another aspect, a system for loading an intravenous (IV) set is provided. The system includes an IV tubing fitment and a fluid delivery device. The fluid delivery device includes a body forming, in part, a mating recess and a door forming another part of the mating recess. While the door is in an open position, the mating recess is configured to receive the IV tubing fitment such that the mating recess is uniformly mated with a substantially hemispherical surface of the IV tubing fitment, and a portion of the IV tubing fitment is positioned outside of the body of the fluid delivery device. When the door is moved to a closed position while the IV tubing fitment is mated with the mating recess, the door is configured to couple with the body of the fluid delivery device enclosing the IV tubing fitment in a pocket. In some implementations, the door includes one or more coupling members configured to couple to a portion of the IV tubing fitment (e.g., an outer rim of the uniform body). In some implementations, the door is prevented from coupling with the body of the fluid delivery device while at least a portion of the IV tubing fitment is misloaded.

In another aspect, a method for loading an intravenous (IV) set to a fluid delivery device is provided. The method is performed at a fluid delivery device including a body forming, in part, a mating recess and a door forming another part of the mating recess. The method includes while the door is in an open position, receiving an IV tubing fitment at the mating recess such that a substantially hemispherical surface of the IV tubing fitment is uniformly mated with a substantially identical surface of the mating recess. The method further includes closing the door such that the door couples with the body of the fluid delivery device and forms an enclosure that is configured to surround at least a portion of the IV tubing fitment.

The foregoing and other features, aspects and advantages of the disclosed implementations will become more apparent from the following detailed description and accompanying drawings.

### BRIEF DESCRIPTION OF THE DRAWINGS

The accompanying drawings, which are included to provide further understanding of the disclosure and are incorporated in and constitute a part of this specification, illustrate implementations of the disclosure and together with the description serve to explain the principles of the disclosure.
Figure 1 shows a patient care system, in accordance with various aspects of the present disclosure.
Figure 2 shows an infusion pump, in accordance with various aspects of the present disclosure.
Figure 3 illustrates an IV tubing fitment, in accordance with some implementations.
Figures 4A-4C illustrate different views of the IV tubing fitment in accordance with some implementations.
Figure 5 illustrates an enclosure of a fluid delivery device, in accordance with some implementations.
Figures 6A-6D illustrate the loading of an IV tubing fitment into a fluid delivery device, in accordance with some implementations.
Figure 7 provides a cross-sectional view of an IV tubing fitment mated with at mating recess of a fluid delivery device, in accordance with some implementations.
Figure 8 illustrates a view of a door of a fluid delivery device, in accordance with some implementations.
Figure 9 illustrates a flow diagram of a method for loading an IV set to a fluid delivery device, in accordance with some implementations.

Like reference numerals refer to corresponding parts throughout the drawings.

### DETAILED DESCRIPTION

Embodiments of the invention are defined by the appended claims. In the following, parts of the description and drawings referring to embodiments which are not covered by the claims are not presented as embodiments of the invention, but as examples useful for understanding the invention. The disclosed IV tubing fitment secures an IV set to fluid delivery device such that a tubing of the IV set cannot be damaged or altered by misloading of the IV set. The IV tubing fitment includes an intuitive design that allows users to readily recognize an orientation in which the IV tubing fitment should be placed within a fluid delivery device to ensure that the IV set is properly loaded. Further, the IV tubing fitment is designed such that the fluid delivery device is unable to close while the IV tubing fitment is misloaded. The IV tubing fitment reduces the chances of the IV set being damaged by being forced into a fluid delivery device thereby improving the accuracy and consistency of the flow rate generated by the fluid delivery device. The detailed description set forth below is intended as a description of various configurations of the subject technology and is not intended to represent the only configurations in which the subject technology may be practiced. The detailed description includes specific details for the purpose of providing a thorough understanding of the subject technology. However, it will be apparent to those skilled in the art that the subject technology may be practiced without these specific details. In some instances, well-known structures and components are shown in block diagram form in order to avoid obscuring the concepts of the subject technology. Like components are labeled with identical element numbers for ease of understanding. Reference numbers may have letter suffixes appended to indicate separate instances of a common element while being referred to generically by the same number without a suffix letter.

While the following description is directed to an IV tubing fitment configured to be loaded into a fluid delivery device, it is to be understood that this description is only an example of usage and does not limit the scope of the claims. Various aspects of the disclosed IV tubing fitment may be used in any application where it is desirable to secure an IV set or other type of tubing to a fluid delivery device.

The disclosed IV tubing fitment overcome several challenges discovered with respect to certain conventional IV sets. One challenge with certain conventional IV sets is the misloading of IV sets that can cause damage to a tube of the IV sets resulting in inaccurate, inconsistent, and uncontrollable flow rates. Because of the inaccuracies or the inconsistencies of caused by misloaded IV sets, thereby potentially harming the patient or decreasing the efficacy of a treatment, the use of conventional IV sets afford limited protection from adverse conditions due to misloading.

Therefore, in accordance with the present disclosure, it is advantageous to provide an IV tubing fitment as described herein that eliminates or substantially reduces the possibility of misloading IV sets. The disclosed IV tubing fitment securely holds an IV set in place and also prevents a fluid delivery device from receiving an IV set if misloaded thereby improving the accuracy and consistency of a generated flow rate.

An example of an IV tubing fitment is now described.

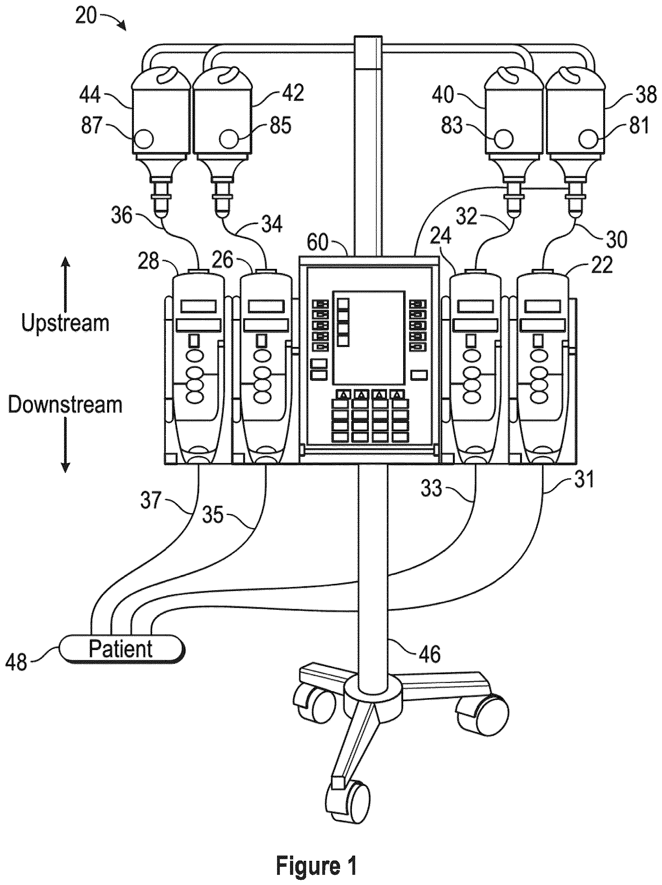

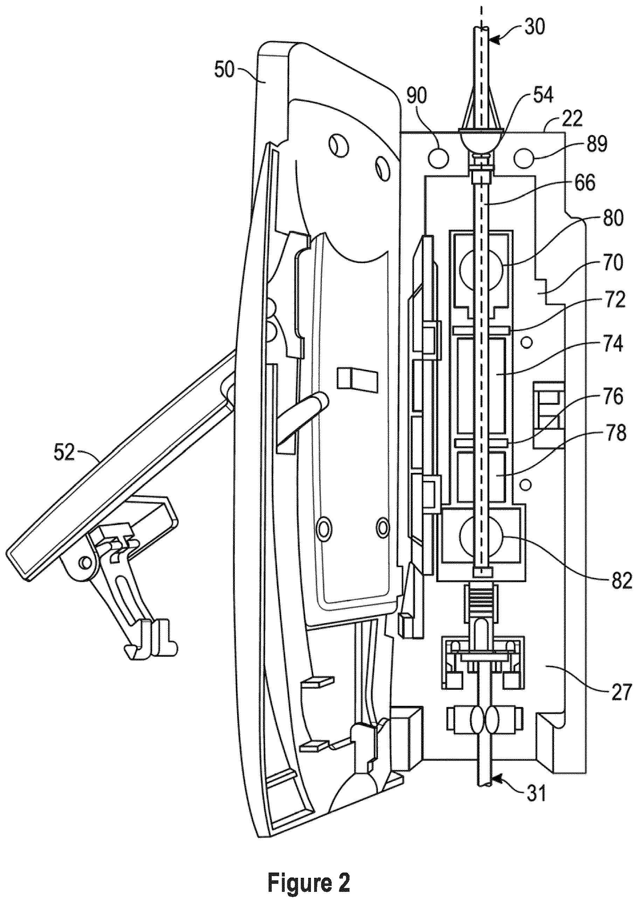

Figure 1 shows a patient care system, in accordance with various aspects of the present disclosure. The patient care system 20 includes four infusion pumps 22, 24, 26, and 28 each of which is fluidly connected with an upstream fluid line 30, 32, 34, and 36, respectively. Each fluid line 30, 32, 34, and 36 is held in place at a respective infusion pumps 22, 24, 26, and 28 via a respective IV tubing fitment (e.g., IV tubing fitment 54; Figure 2). Each of the four infusion pumps 22, 24, 26, and 28 is also fluidly connected with a downstream fluid line 31, 33, 35, and 37, respectively. The fluid lines can be any type of fluid conduit, such as an IV administration set, through which fluid can flow through. It should be appreciated that any of a variety of pump mechanisms can be used including syringe pumps.

Fluid supplies 38, 40, 42, and 44, which may take various forms but in this case are shown as bottles, are inverted and suspended above the pumps. Fluid supplies may also take the form of bags or other types of containers including syringes. Both the patient care system 20 and the fluid supplies 38, 40, 42, and 44 are mounted to a roller stand, IV pole 46, tabletop, etc.

A separate infusion pump 22, 24, 26, and 28 is used to infuse each of the fluids of the fluid supplies into the patient. The infusion pumps are flow control devices that will act on the respective fluid line to move the fluid from the fluid supply through the fluid line to the patient 48. Because individual pumps are used, each can be individually set to the pumping or operating parameters required for infusing the particular medical fluid from the respective fluid supply into the patient at the particular rate prescribed for that fluid by the physician. Such medical fluids may include drugs or nutrients or other fluids. The infusion pumps 22, 24, 26, and 28 are controlled by a controller 60. In some implementations, the controller 60 is communicatively coupled to memory storing one or more instructions for operating the infusion pumps 22, 24, 26, and 28 and/or other collected data as described below.

Fluid supplies 38, 40, 42, and 44 are each coupled to an electronic data tag 81, 83, 85, and 87, respectively, or to an electronic transmitter. Any device or component associated with the infusion system may be equipped with an electronic data tag, reader, or transmitter.

Typically, medical fluid administration sets have more parts than are shown in Figure 1. Many have check valves, drip chambers, valves with injection ports, connectors, and other devices well known to those skilled in the art. These other devices have not been included in the drawings so as to preserve clarity of illustration.

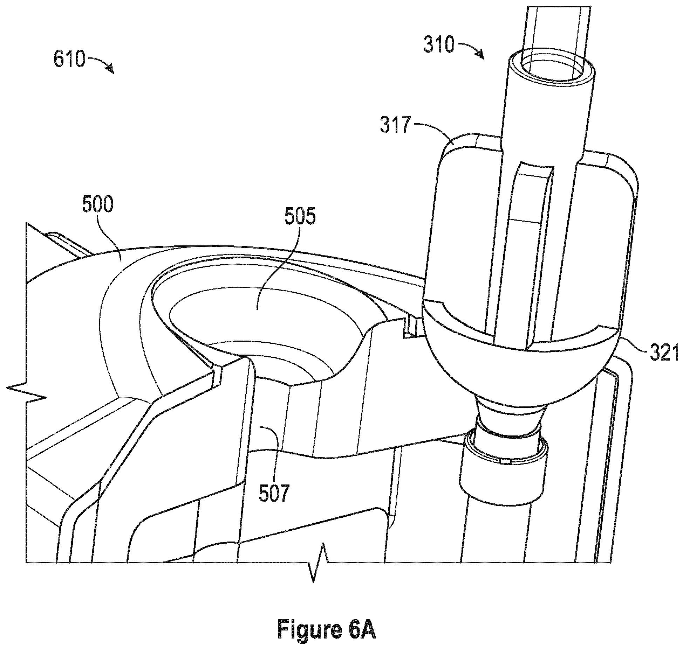

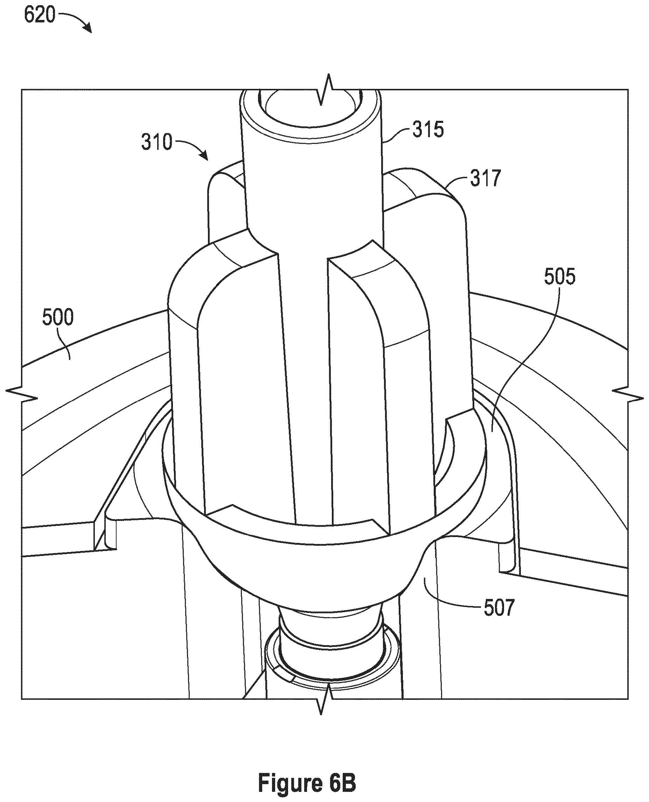

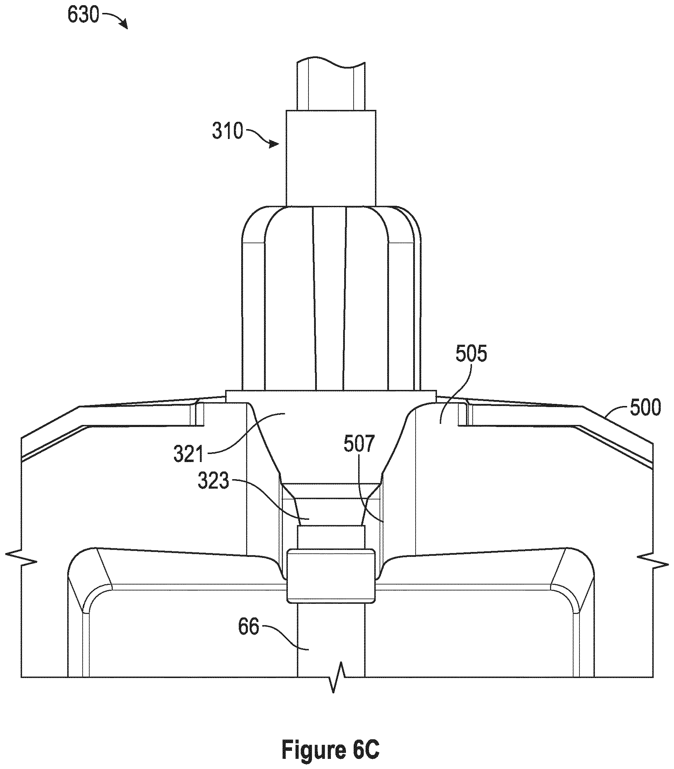

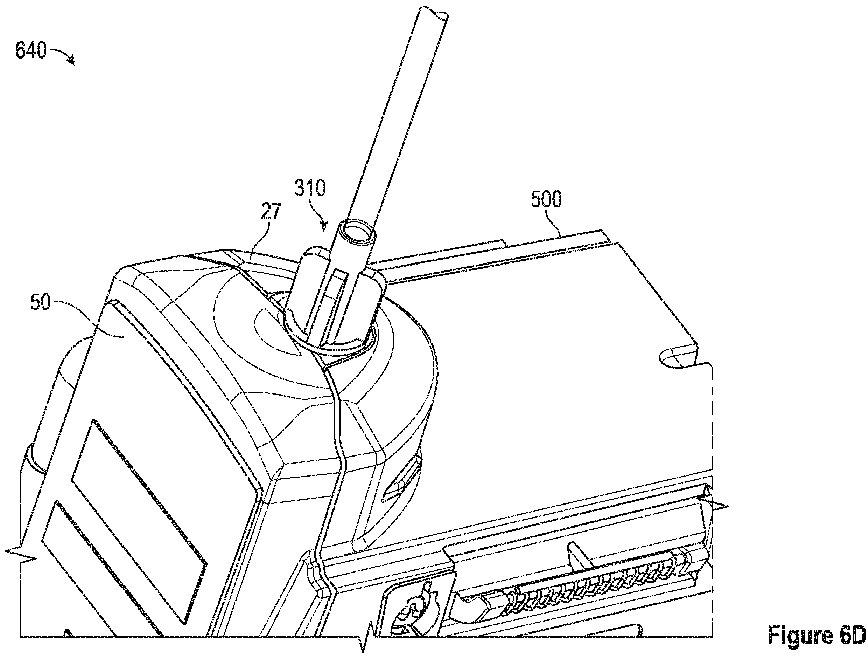

Turning now to Figure 2, an infusion pump 22 having a body 27 is shown in perspective view, in accordance with various aspects of the present disclosure. The infusion pump 22 is shown with the front door 50 open, showing the upstream fluid line 30 and downstream fluid line 31 in operative engagement with the infusion pump 22. More specifically, the fluid line 30 is coupled to (or received by) an IV tubing fitment 54 that is held in place within a pocket and/or mating recess of the infusion pump 22. The IV tubing fitment 54 is a unitary body including a first end configured to couple to a first portion of a tubing (e.g., tube 66) and a second end opposite the first end coupled to (or configured to receive) a second portion of the tubing (e.g., fluid line 30). The IV tubing fitment 54 includes a substantially hemispherical surface that, when received by the infusion pump 22, is uniformly mated with a substantially identical surface of the mating recess of the infusion pump 22. Additional information on the placement of the IV tubing fitment 54 is provided below in reference to Figures 6A-6D.

The IV tubing fitment 54 reduces the chances of an IV set (e.g., at least the upstream fluid line 30, the tube 66, and the downstream fluid line 31) from being misloaded by preventing the door 50 of the infusion pump 22 from closing if the substantially hemispherical surface of the IV tubing fitment 54 is not mated to the mating recess of the infusion pump 22. For example, a diameter of the substantially hemispherical surface of the IV tubing fitment 54 is such that the door 50 cannot be closed unless the IV tubing fitment 54 is in the correct position (i.e., the substantially hemispherical surface of the IV tubing fitment 54 is mated to the mating recess). More specifically, in some implementations, the substantially hemispherical surface of the IV tubing fitment 54 has a diameter that is substantially equal to a diameter of the mating recess of the infusion pump 22. The diameter of the substantially hemispherical surface of the IV tubing fitment 54 prevents the infusion pump 22 from closing when the substantially hemispherical surface of the IV tubing fitment 54 is not uniformly mated with the substantially identical surface of the mating recess of the infusion pump 22. In some implementations, a hook on the door 50 latching arm 52 will not engage the latch hook unless the IV tubing fitment 54 is in the correct position. Thus, by not allowing engagement, the IV tubing fitment 54 prevents a user from forcing the latch to close the door 50 and applying a forced leverage, which can cause damage to the IV set, the door hinges, or breakage of the platen.

Additionally, the IV tubing fitment 54 allows the IV set to rotate about its longitudinal axis (e.g., center line of the upstream fluid line 30) while preventing the IV set from twisting, kinking, and/or entangling. In some implementations, the IV tubing fitment 54 rotates about a longitudinal axis at least 270 degrees in a clockwise and counterclockwise direction. In some implementations, the IV tubing fitment 54 rotates about the longitudinal axis a full 360 degrees in a clockwise and counterclockwise direction. By preventing the IV set from twisting, kinking, and/or entangling, the IV tubing fitment 54 improves the accuracy and consistency of the flow rate be eliminating or reducing any accidental or unintentional occlusion caused by movement of the IV set, as well as potential damage to the IV set. Further, the IV tubing fitment 54 is further configured to center the tubing 66 when the substantially hemispherical surface of the IV tubing fitment 54 is mated to the mating recess of the infusion pump 22.

The infusion pump 22 directly acts on the tube 66 that connects the upstream fluid line 30 to the downstream fluid line 31 to form a continuous fluid conduit, extending from the respective fluid supply 38 (Figure 1) to the patient 48, through which fluid is acted upon by the pump to move fluid downstream to the patient. Specifically, a pumping mechanism 70 acts as the flow control device of the pump to move fluid though the conduit. The upstream and downstream fluid lines and/or tube 66 may be coupled to a pump cassette or cartridge that is configured to be coupled to the pump 22.

The type of pumping mechanism may vary and may be for example, a multiple finger pumping mechanism. For example, the pumping mechanism may be of the "four finger" type and includes an upstream occluding finger 72, a primary pumping finger 74, a downstream occluding finger 76, and a secondary pumping finger 78. The "four finger" pumping mechanism and mechanisms used in other linear peristaltic pumps operate by sequentially pressing on a segment of the fluid conduit by means of the cam-following pumping fingers and valve fingers 72, 74, 76, and 78. The pressure is applied in sequential locations of the conduit, beginning at the upstream end of the pumping mechanism and working toward the downstream end. At least one finger is always pressing hard enough to occlude the conduit. As a practical matter, one finger does not retract from occluding the tubing until the next one in sequence has already occluded the tubing; thus, at no time is there a direct fluid path from the fluid supply to the patient. The operation of peristaltic pumps including four finger pumps is well known to those skilled in the art and no further operational details are provided here.

Figure 2 further shows a downstream pressure sensor 82 included in the pump 22 at a downstream location with respect to the pumping mechanism. The downstream pressure sensor 82 is mounted to the flow control device 70 and is located adjacent and downstream in relation to the flow control device. The downstream pressure sensor is located downstream from the flow control device, that is, at a location between the patient 48 (Figure 1) and the flow control device, so that the connection of the correct fluid supply with the correct pump may be verified before any fluid is pumped to the patient.

With reference still to Figure 2, an upstream pressure sensor 80 may also be included in the pump 22. The upstream pressure sensor is assigned to the flow control device or pumping mechanism 70 and, in this implementation, is further provided as an integral part of the pump 22. It is mounted to the flow control device 70 and is located adjacent and upstream in relation to the flow control device. The upstream pressure sensor is located upstream from the flow control device, that is, at a location between the fluid supply 38 (Figure 1) and the flow control device, so that the connection of the correct fluid supply with the correct pump may be verified before any fluid is pumped to the patient.

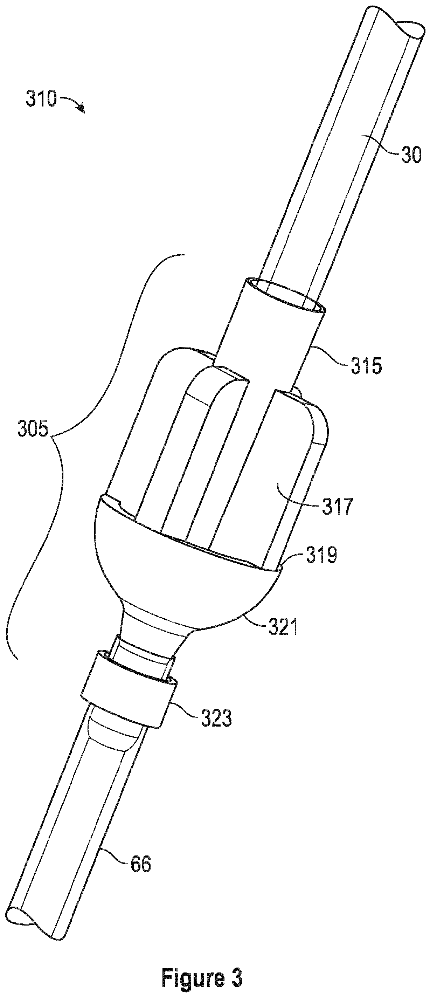

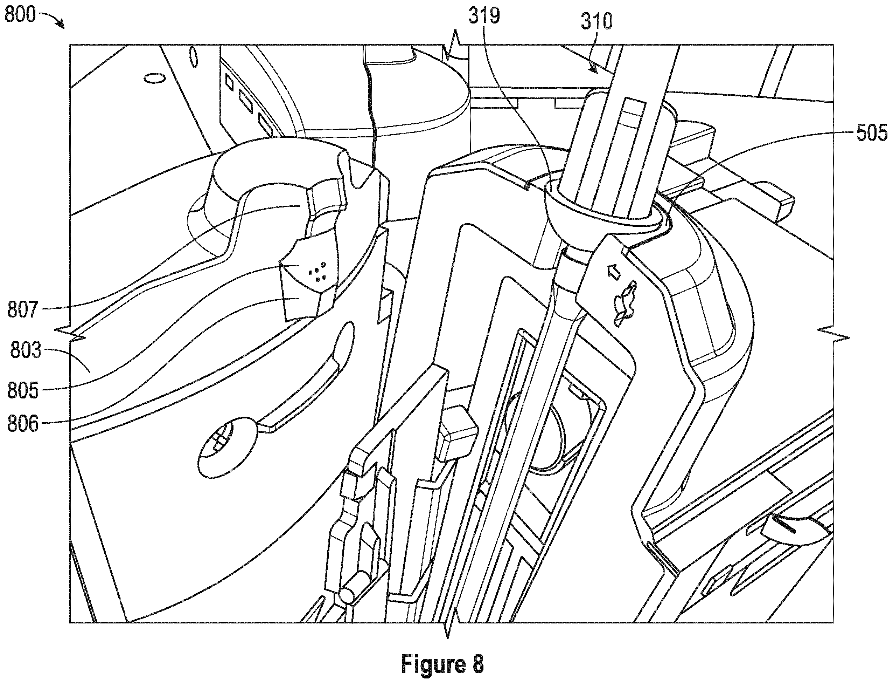

The pump 22 or a portion of the pump 22 may also be equipped with an electronic data tag or data transmitter. For example, as shown in Figure 2, pump 22 may be equipped with a data tag 89 or a reader device 90 for providing or receiving infusion data. The data reader devices may include RFID readers (or receivers) or other wireless devices that are compatible with the data tags associated with the fluid containers. A data transmitter may transmit interrogation signals to the electronic data tags 81, 83, 85, 87 associated with the fluid containers for obtaining infusion data from those tags. Although referred to as data transmitting devices or RFID tags or RFID transponders, data transmitting devices may also receive or read data and may also be writable. Typically, medical tubing is a disposable product that is used once and then discarded. The medical tubing may be formed from any suitable material, e.g., soft PVC, silicone, thermoplastic vulcanizate (TPV) (ethylene propylene diene monomer (EPDM) + polypropylene (PP)), thermoplastic polyurethane (TPU), thermoplastic styrenic elastomer (TPS) (styrene-butadienestyrene (SBS) /styrene-ethylene-butylene-styrene (SEBS) /styrene-isoprene-rubber (SIS) /styrene-ethylene-propylene-styrene (SEPS)) and its blending with polyolefin , thermoplastic polyester elastomer (TPEE) (polyether ester) rubber). As shown in Figure 2, medical tubing 66 may be inserted into or otherwise engaged by pump 22. Infusion pump 22 may include any of large volume (e.g., at least 100 milliliter of fluid from a single container), patient-controlled analgesia (PCA), ambulatory pump or insulin pump that drive tubing segment(s) to deliver medication or nutrients into a patient's body in controlled amounts. The medical tubing 66 is compressed when the pump door 50 is closed. With the pump door 50 closed, the medical tubing 66 is constrained within the infusion pump 22 mating recess (by using the IV tubing fitment 54) and directly contacted by the upstream force sensor 80. As discussed above, there are many sources of variation in measuring the force on the medical tubing 66 by the sensor 80. Figure 3 illustrates an IV tubing fitment, in accordance with some implementations. The IV tubing fitment 310 is an example of the IV tubing fitment 54 described above in reference to Figure 2. The IV tubing fitment 310 includes a unitary body 305. In some implementations, the unitary body 305 is molded. The unitary body 305 includes a first end 323 configured to couple to a first portion of a tubing (e.g., tube 66; Figure 2), a substantially hemispherical surface 321 adjacent to the first end 323, and a cylindrical body 315 adjacent to the substantially hemispherical surface 321 and configured to receive a second portion of the tubing (e.g., upstream fluid line 30; Figure 2). The substantially hemispherical surface 321 is positioned between the first end 323 and the cylindrical body 315. The cylindrical body 315 further includes a plurality of wings 317 extending from the cylindrical body 315 towards outer rim 319. The plurality of wings 317 provide a user an intuitive means of determining the portion of the IV tubing fitment 310 that should be placed within a fluid delivery device (e.g., infusion pump 22; Figure 2). In some implementations, the plurality of wings 317 do not reach the outer rim 319 allowing the IV tubing fitment 310 to couple with the fluid delivery device restricting the IV tubing fitment 310 movement in the longitudinal and latitudinal directions while also allowing the IV tubing fitment 310 to rotate about the longitudinal axis (i.e., the plurality of wings 317 do not engage or make contact the fluid delivery device). More specifically, the outer rim 319 is configured to engage with a door of the fluid delivery device such that when the outer rim 319 is engaged with the door of the fluid delivery device, movement of the unitary body 305 is restricted in at least one of a longitudinal and a latitudinal directions as described below in reference to Figure 8.

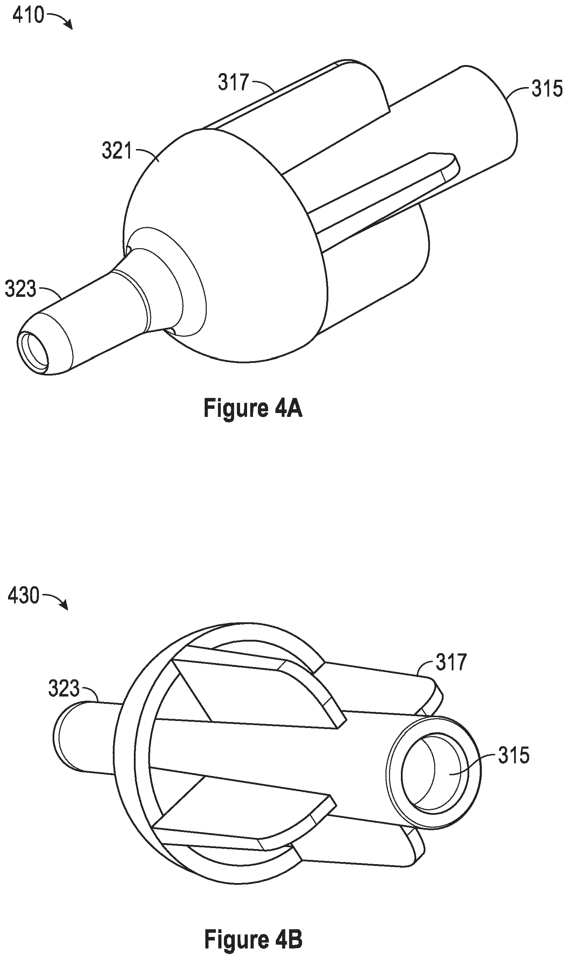

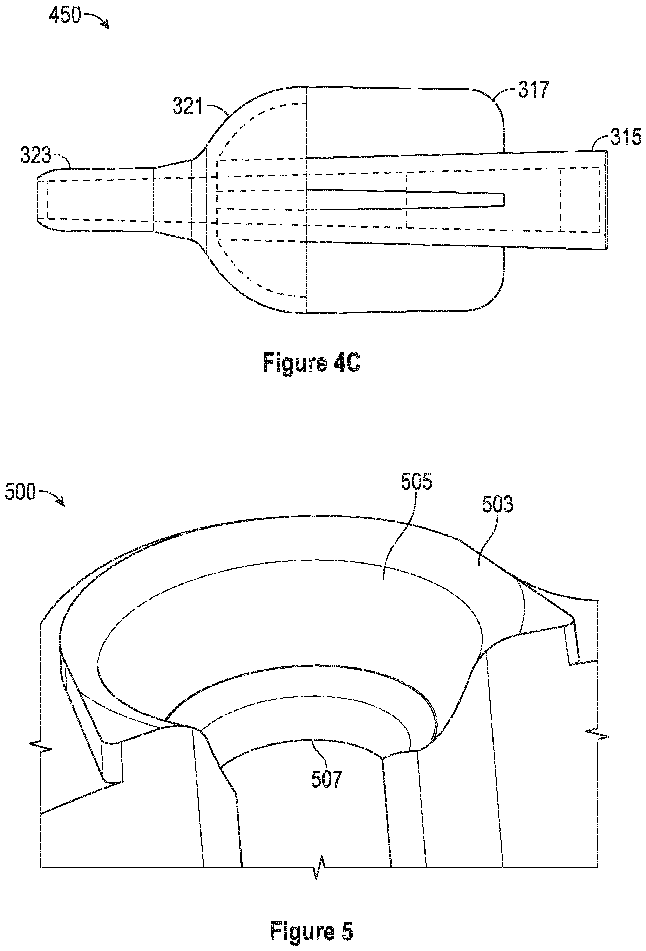

As described in detail below in reference to Figures 6A-6D, the unitary body 305 is configured to be received into a mating recess of a fluid delivery device such that the substantially hemispherical surface 321 is uniformly mated with a substantially identical surface of the mating recess of the fluid delivery device. In some implementations, the substantially hemispherical surface 321 is uniformly mated with the substantially identical surface of a mating recess 505 (Figure 5) only when the first end 323 of the IV tubing fitment 310 is received within a pocket 507 of the mating recess 505 preventing the IV tubing fitment 310 from being misloaded. Figures 4A-4C illustrate different views of the IV tubing fitment 310 (Figure 3) in accordance with some implementations. A first view 410 provides a front view of the IV tubing fitment 310. The first view 410 shows the unitary body 305 (Figure 3) including a first end 323, a substantially hemispherical surface 321 adjacent to the first end 323, and a cylindrical body 315 adjacent to the substantially hemispherical surface 321. In some implementations, the first end 323 is a nozzle configured to be fitted inside a tube 66 (Figure 2). In some implementations, once the first end 323 has been fitted inside the tube 66, the tube 66 and the first end 323 are coupled such that they cannot be separated accidentally unless excessive force is applied. Excessive force, for purposes of this disclosure means, a force greater than accidental tugs and pulls and forces greater than those applied by the fluid delivery device. In some implementations, the first end 323 is molded within a portion of the tube 66 (when the unitary body 305 is molded). As described above, the cylindrical body 315 includes a plurality of wings 317 that extend from the center of the cylindrical body 315 towards an outer rim 319 (Figure 3). A second view 430 provides a rear view of the IV tubing fitment 310. The second view 430 shows the unitary body 305 including the first end 323, the cylindrical body 315, and the plurality of wings 317 coupled to the cylindrical body 315. In some implementations, the cylindrical body 315 includes an opening configured to receive an upstream fluid line 30 (Figure 2). In some implementations, the cylindrical body 315 receives the upstream fluid line 30 with a sufficient amount of force to prevent the cylindrical body 315 from being separated from the upstream fluid line 30 accidentally unless excessive force is applied. In some implementations, the cylindrical body 315 is molded over a portion of the upstream fluid line 30 (when the unitary body 305 is molded). By molding the cylindrical body 315 over a portion of the upstream fluid line 30, the portion of the upstream fluid line 30 is prevented from accidentally being pulled out unless excessive force is applied.

A third view 450 provides a side view of the IV tubing fitment 310. The third view 450 shows the unitary body 305 including the first end 323, the substantially hemispherical surface 321 adjacent to the first end 323, the cylindrical body 315 adjacent to the substantially hemispherical surface 321, and the plurality of wings 317. The first, second, and third views 410, 430, and 450 illustrate that the unitary body 305 includes a constant wall thickness, draft angles, and drafts to promote easy and efficient moldability.

Figure 5 illustrates an enclosure of a fluid delivery device, in accordance with some implementations. Fluid delivery device 500 is an instance of any infusion pump described above in reference to Figures 1 and 2. The fluid delivery device 500 includes a bezel housing 503 including a mating recess 505, and a pocket 507 on a portion of the fluid delivery device body (e.g., body 27; Figure 2). The pocket 507 is positioned at a center of the mating recess 505. The mating recess 505 of a fluid delivery device includes a hemispherical surface substantially identical to a substantially hemispherical surface 321 of the IV tubing fitment 310 (Figures 3-4C) and configured to mate with the IV tubing fitment 310. More specifically, in some implementations, the mating recess 505 has a concave surface that is configured to mate with a convex surface of the IV tubing fitment 310. The mating recess 505 does not include any edges or steps on its mating surface, thus preventing IV tubing fitment 310 from being misloaded.

In some implementations, the substantially hemispherical surface 321 of the uniform body 305 is uniformly mated with the (substantially identical) surface of the mating recess 505 only when the first end 323 of the unitary body 505 is received within the pocket 507 of the mating recess 505, which prevents the IV tubing fitment from being misloaded. In some implementations, when the first end 323 of the unitary body 305 is received within the pocket 507 of the mating recess 505, the first portion of the tubing (e.g., tube 66; Figure 2) coupled with the first end 323 of the unitary body is aligned along a center of the mating recess 505.

In some implementations, portions of the mating recess 505 and pocket 507 are formed by the body of the fluid delivery device 500 and additional portions of the mating recess and the pocket (e.g., additional mating recess 805 and additional pocket 806; Figure 8) are formed by a fluid delivery device door (e.g., door 50; Figure 2). Additional detail on the additional mating recess 805 and the additional pocket 806 is provided below in reference to Figure 8.

Figures 6A-6D illustrate the loading of an IV tubing fitment into a fluid delivery device, in accordance with some implementations. The IV tubing fitment and fluid delivery device described in reference to Figures 6A-6D can be any IV tubing fitment and fluid delivery device described above in reference to Figures 1-5.

A first operational view 610 shows an IV tubing fitment 310 being positioned within a mating recess 505 and a pocket 507 (at the center of the mating recess 505) of a fluid delivery device 500 (Figure 5). In particular, while a door 50 (Figure 2) of the fluid delivery device 500 is open, the mating recess 505 of the fluid delivery device 500 is configured to receive the IV tubing fitment 310. As shown in the first operation view 610, the IV tubing fitment 310 is configured such that the fluid delivery device 500 can only receive the IV tubing fitment 310 in a single configuration. More specifically, the substantially hemispherical surface of the IV tubing fitment 310 is uniformly mated with the substantially identical surface of the mating recess 505 only when the first end 323 of the IV tubing fitment 310 is received within the pocket 507 of the mating recess 505 preventing the IV tubing fitment 310 from being misloaded. For example, if the user attempted to mate the substantially hemispherical surface of the IV tubing fitment 310 with the substantially identical surface 321 of the mating recess 505 without positioning the first end 323 of the IV tubing fitment 310 within the pocket 507, the substantially hemispherical surface 321 and the mating recess 505 would not be mated (which would not allow for the door 50 of the fluid delivery device 500 to close). Further, if the user does not place the first end 323 of the IV tubing fitment 310 within the pocket 507 of the mating recess 505, the door 50 of the fluid delivery device 500 would not be able to close.

Additionally, if a user attempted to place a portion of the IV tubing fitment 310 including the plurality of wings 317 within the mating recess 505, the IV tubing fitment 310 would not securely fit in the fluid delivery device 500 and, as described above, the door 50 of the fluid delivery device 500 would be prevented from closing. In addition to preventing the door 50 of the fluid delivery device 500 from closing, the plurality of wings 317 is further configured to operate as an intuitive indicator of the appropriate placement of the IV tubing fitment 310. As described above in reference to Figure 5, the mating recess 505 has no edges or steps that allow the IV tubing fitment 310 to be misloaded (e.g., via the plurality of wings 317 or other surface of the IV tubing fitment 310).

In some implementations, the substantially hemispherical surface 321 of the IV tubing fitment 310 and the substantially identical surface of the mating recess 505 include visual indicators of how the IV tubing fitment 310 should be placed in the fluid delivery device 500. For example, in some implementations, the substantially hemispherical surface 321 of the IV tubing fitment 310 and the substantially identical surface of the mating recess 505 can be the same color (e.g., green) indicating to users to match the colors. In some implementations, the hemispherical surfaces on the IV tubing fitment 310 and the mating recess 505 allow easier cleaning of the IV tubing fitment 310, the mating recess 505 of fluid delivery device 500, and the door 50 of the fluid delivery device 500.

A second operational view 620 shows the placement of the IV tubing fitment 310 within the mating recess 505 of the fluid delivery device 500. In particular, the IV tubing fitment 310 is received by the mating recess 505 such that the mating recess 505 is uniformly mated with the substantially hemispherical surface 321. In some implementations, a portion of the IV tubing fitment 310 is positioned outside of the body 27 (Figure 2) of the fluid delivery device 500. For example, when the IV tubing fitment 310 is positioned in the mating recess 505, the IV tubing fitment 310 cylindrical body 315 and plurality of wings 317 are positioned are outside of the mating recess 505 and, more specifically, outside of the body 27 of the fluid delivery device 500. A third operational view 630 provides another view of the placement of the IV tubing fitment 310 within the mating recess 505 of the fluid delivery device 500. The third view 630 illustrates that the substantially hemispherical surface 321 of the IV tubing fitment 310 is configured to self-center a tube 66 (Figure 2). More specifically, when the first end 321 of the IV tubing fitment 310 is received within the pocket 507 of the mating recess 505, the first portion of the tubing (i.e., tube 66) is aligned along a center of the mating recess 505.

A fourth operational view 640 show the fluid delivery device 500 with its door 50 closed. In particular, when the door 50 of the fluid delivery device 500 is closed an enclosure for the IV tubing fitment 310 is formed. While the IV tubing fitment 310 is mated with the mating recess 505, the door 50 engages and couples with the body 27 of the fluid delivery device 500 enclosing a portion the IV tubing fitment 310 (e.g., at least the substantially hemispherical surface 321 and the first end 323 of the IV tubing fitment 310). While the portion of the IV tubing fitment 310 is enclosed by the fluid delivery device 500 , the IV tubing fitment 310 is unable to be moved in longitudinal and latitudinal directions (i.e., the IV tubing fitment 310 cannot be moved to the left or right, or pulled upward or pushed downwards from its position in the mating recess 505. In some implementations, although the portion of the IV tubing fitment 310 is enclosed by the fluid delivery device 500, the IV tubing fitment 310 is free to rotate about a longitudinal axis while keeping the IV set from kinking, twisting, and/or entangling. In some implementations, the portion of the IV tubing fitment 310 can rotate a full 360 degrees while enclosed by the fluid delivery device 500.

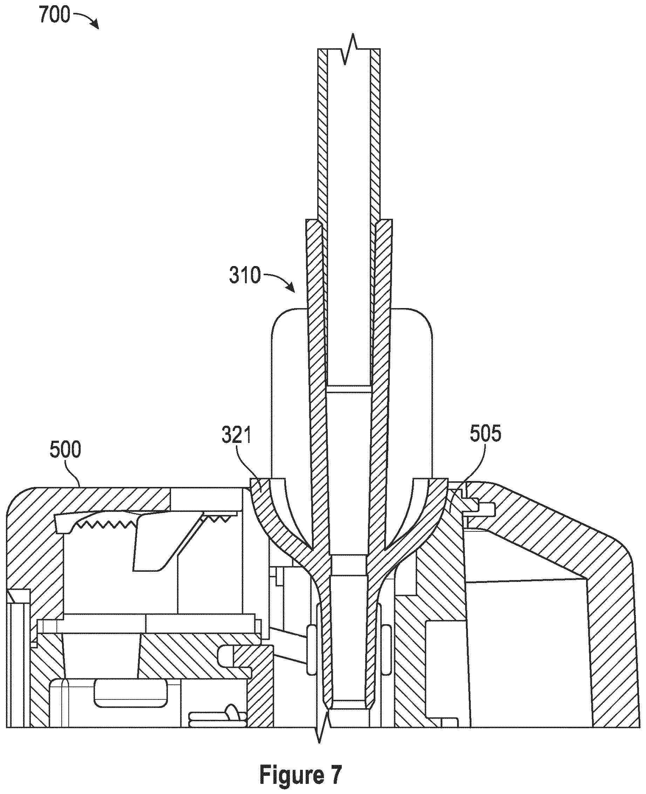

Figure 7 provides a cross-sectional view 700 of an IV tubing fitment mated with at mating recess of a fluid delivery device, in accordance with some implementations. The IV tubing fitment and fluid delivery device shown in cross-sectional view 700 can be any IV tubing fitment the fluid delivery device described above in reference to Figures 1-6D. The cross-sectional view 700 includes IV tubing fitment 310 mated with the mating recess 505 of the fluid delivery device 500. As shown in cross-sectional view 700 when the IV tubing fitment 310 is mated with the mating recess 505 of the fluid delivery device 500, an IV set (e.g., at least an upstream fluid line 30, an tube 66, and a downstream fluid line 31; Figure 2) is centered along a longitudinal axis such that the IV set cannot twist, entangle, or kink. Further, the positioning of the IV tubing fitment 310 within the mating recess 505 of the fluid delivery device 500 and its ability to rotate about the longitudinal axis reduces the number of unintentional pulls or forces experienced by the IV set.

Figure 8 illustrates a view of a door of a fluid delivery device, in accordance with some implementations. View 800 shows a fluid delivery device (e.g., infusion pump 22; Figure 2) including a door 803, a mating recess 505, a pocket 507, and an IV tubing fitment 310 mated to the mating recess 505 of the fluid delivery device 500 as described above in reference to Figures 1-7. The door 803 is an example of the door 50 described above in reference to Figure 2.

In some implementations, the door 503 forms another portion of the mating recess and pocket of the fluid delivery device (e.g., additional mating recess 805 and additional pocket portion 806). In some implementations, when the door 803 is closed while the IV tubing fitment 310 (Figure 3) is mated with the mating recess 505 (Figure 5), the additional mating recess 805 also mates with the IV tubing fitment 310. In some implementations, for the door 803 to close, the additional mating recess 805 must also be mated with the IV tubing fitment 310. For example, if either the additional mating recess 805 or the mating recess 505 are not mated with the substantially hemispherical surface 321 of the IV tubing fitment 310, the door 803 is prevented from closing as described above. Similarly, if the first end 323 of the IV tubing fitment 310 is not received within the additional pocket portion 806 the door 803 is also prevented from closing. In this way, the IV tubing fitment 310 is not misloaded.

In some implementations, the door 803 includes one or more coupling members 807 configured to engage with a portion of the IV tubing fitment 310. In some implementations, the coupling members 807 include teeth, hooks, clasps, levers, etc. In some implementations, the one or more coupling members 807 of the door 803 are configured to engage with an outer rim 319 of the IV tubing fitment 310. In some implementations, if one or more coupling members 807 of the door 803 are unable to engage with the outer rim 319 of the IV tubing fitment 310, the door 50 is prevented from closing. In some implementations, the one or more coupling members 807 engage with the outer rim 319 of the IV tubing fitment 310 such that a portion of the IV tubing fitment 310 (e.g., at least the substantially hemispherical surface 321 and the first end of the IV tubing fitment 310) is enclosed by fluid delivery device 500 (Figure 5). More specifically, the one or more coupling members 807 form a lid or an enclosure with narrower diameter (with respect to a diameter of the substantially hemispherical surface 321) such that the IV tubing fitment 310 cannot be pulled from the fluid delivery device 500 while still allowing an IV set (Figure 2) to rotate about a longitudinal axis.

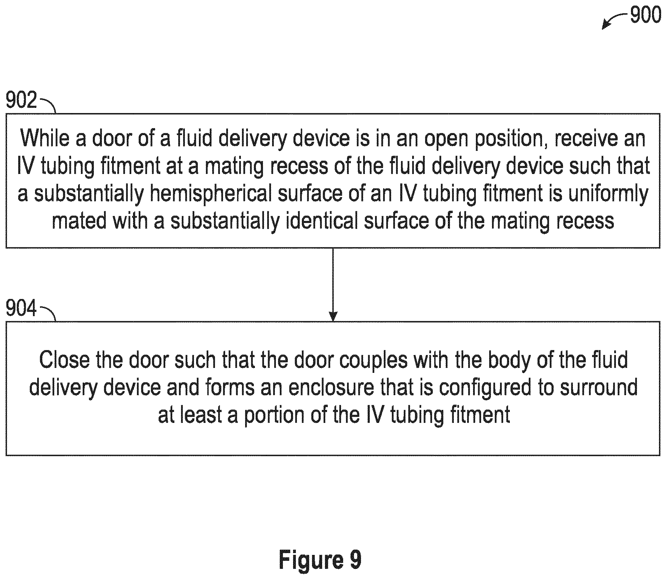

Figure 9 illustrates a flow diagram of a method for loading an IV set to a fluid delivery device, in accordance with some implementations. Method 900 can be performed using any IV tubing fitment and fluid delivery device described above in reference to Figures 1-8. The method 900 includes while a door of a fluid delivery device is in an open position, receiving (902) an IV tubing fitment at a mating recess of the fluid delivery device such that a substantially hemispherical surface of an IV tubing fitment is uniformly mated with a substantially identical surface of the mating recess. The method 900 further includes closing (904) the door such that the door couples with the body of the fluid delivery device and forms an enclosure that configured to surrounds at least a portion of the IV tubing fitment. The enclosure restricts the lateral and longitudinal movement of an IV tubing fitment within the fluid delivery device while also allowing the IV tubing fitment to rotate about the longitudinal axis.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

In one aspect, the subject technology may be implemented without utilizing some of the components, elements, functions or operations described herein. In one aspect, the subject technology may be implemented utilizing additional components, elements, functions or operations.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An implementation may provide one or more examples. A phrase such an implementation may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

In one aspect, the term "coupled," or the like may refer to being directly coupled. In another aspect, the term "coupled," or the like may refer to being indirectly coupled.

Terms such as "top," "bottom," "front," "rear" and the like if used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Various items may be arranged differently (e.g., arranged in a different order, or partitioned in a different way) all without departing from the scope of the subject technology. Moreover, nothing disclosed herein is intended to be dedicated to the public regardless of whether such disclosure is explicitly recited in the claims.

Furthermore, to the extent that the term "include," "have," or the like is used, such term is intended to be inclusive in a manner similar to the term "comprise" as "comprise" is interpreted when employed as a transitional word in a claim.

## Claims

1. An intravenous, IV, tubing fitment (54, 310), comprising:
a unitary body (305) configured to couple with a fluid delivery device (22, 24, 26, 28) including a body (27) forming, in part, a mating recess (505) and a door (50, 803), wherein the door (50, 803) forms part of the mating recess (505), the unitary body (305) comprising:
a first end (323) configured to couple to a first portion (66) of a tubing,
a substantially hemispherical surface (321) adjacent to the first end (323), and
a cylindrical body (315) adjacent to the substantially hemispherical surface (321), the cylindrical body (315) including a plurality of wings (317) and configured to receive a second portion (30) of the tubing; and
wherein the unitary body (305) is configured to be received into the mating recess (505) of the fluid delivery device (22, 24, 26, 28) such that:
the substantially hemispherical surface (321) is configured to be uniformly mated with a substantially identical surface of the mating recess (505),
the cylindrical body (315) is configured to be positioned outside of the mating recess (505),
when the door is moved to a closed position from an open position while the unitary body is not mated with the mating recess, the door is prevented from coupling with the body of the fluid delivery device, and
when the door (50, 803) is moved to the closed position from the open position while the unitary body (305) is mated with the mating recess (505), the unitary body (305) is configured to be enclosed in a pocket (507) of the fluid delivery device (22, 24, 26, 28).

2. The IV tubing fitment (54, 310) of Claim 1, wherein the unitary body (305), when received by the mating recess (505), is configured to rotate about a longitudinal axis at least 270 degrees in a clockwise and counterclockwise direction.

3. The IV tubing fitment (54, 310) of Claim 1 or Claim 2, wherein the substantially hemispherical surface (321) includes an outer rim (319) that is configured to engage with the door (50, 803) of the fluid delivery device (22, 24, 26, 28).

4. The IV tubing fitment (54, 310) of Claim 3, wherein the outer rim (319), when engaged with the door (50, 803) of the fluid delivery device (22, 24, 26, 28), is configured to restrict movement of the unitary body (305), in at least one of a longitudinal and a latitudinal directions.

5. The IV tubing fitment (54, 310) of any of Claims 1 through 4, wherein the substantially hemispherical surface (321) is configured to be uniformly mated with the substantially identical surface of the mating recess (505) only when the first end (323) of the unitary body (305) is received within the pocket (507) of the mating recess (505) preventing the IV tubing fitment (54, 310) from being misloaded.

6. The IV tubing fitment (54, 310) of Claim 5, wherein when the first end (323) of the unitary body (305) is received within the pocket (507) of the mating recess (505), the first portion (66) of the tubing coupled with the first end (323) of the unitary body (305) is aligned along a center of the mating recess (505).

7. The IV tubing fitment (54, 310) of any of Claims 1 through 6, wherein the substantially hemispherical surface (321) has a diameter that is substantially equal to a diameter of the mating recess (505), wherein the diameter of the substantially hemispherical surface (321) is configured to prevent the fluid delivery device (22, 24, 26, 28) from closing when the substantially hemispherical surface (321) is not uniformly mated with the substantially identical surface of the mating recess (505).

8. The IV tubing fitment (54, 310) of any of Claims 1 through 7, wherein the fluid delivery device (22, 24, 26, 28) is an infusion pump.

9. The IV tubing fitment (54, 310) of any of Claims 1 through 8, wherein the unitary body (305) is molded.

10. A system (20) comprising:
an intravenous, IV, tubing fitment (54, 310) of any of Claims 1 through 6; and
a fluid delivery device (22, 24, 26, 28) comprising a body (27) forming, in part, a mating recess (505) and a door (50, 803) forming another part of the mating recess (505), wherein:
while the door (50, 803) is in an open position, the mating recess (505) is configured to receive the IV tubing fitment (54, 310) such that the mating recess (505) is uniformly mated with a substantially hemispherical surface (321) of the IV tubing fitment (54, 310), and a portion of the IV tubing fitment (54, 310) is positioned outside of the body (27) of the fluid delivery device (22, 24, 26, 28);
when the door is moved to a closed position while the IV tubing fitment is not uniformly mated with the mating recess, the door is prevented from coupling with the body of the fluid delivery device; and
when the door (50, 803) is moved to the closed position while the IV tubing fitment (54, 310) is mated with the mating recess (505), the door (50, 803) is configured to couple with the body (27) of the fluid delivery device (22, 24, 26, 28) enclosing the IV tubing fitment (54, 310) in a pocket (507).

11. The system of Claim 10, wherein the door (50, 803) includes one or more coupling members (807) configured to couple to a portion of the IV tubing fitment (54, 310).

12. The system of Claim 10, wherein the door (50, 803) is configured to be prevented from coupling with the body (27) of the fluid delivery device (22, 24, 26, 28) while at least a portion of the IV tubing fitment (54, 310) is misloaded.

13. A method for loading an intravenous (IV) set to a fluid delivery device (22, 24, 26, 28), the method comprising:
at a fluid delivery device (22, 24, 26, 28) comprising a body (27) forming, in part, a mating recess (505) and a door (50, 803) forming another part of the mating recess (505):
while the door (50, 803) is in an open position, receiving an IV tubing fitment (54, 310) at the mating recess (505) such that a substantially hemispherical surface (321) of the IV tubing fitment (54, 310) is uniformly mated with a substantially identical surface of the mating recess (505);
while the unitary body is not uniformly mated with the mating recess, preventing the door from coupling with the body of the fluid delivery device; and
closing the door (50, 803) such that the door (50, 803) couples with the body (27) of the fluid delivery device (22, 24, 26, 28) and forms an enclosure that is configured to surround at least a portion of the IV tubing fitment (54, 310),
wherein IV tubing fitment (54, 310) comprises:
a unitary body (305) with a first end (323) configured to couple to a first portion (66) of a tubing, wherein the substantially hemispherical surface (321) is adjacent to the first end (323), and a cylindrical body (315) adjacent to the substantially hemispherical surface (321), the cylindrical body (315) including a plurality of wings (317) and configured to receive a second portion (30) of the tubing,
wherein the receiving the IV tubing fitment (54, 310) at the mating recess (505) comprises:
receiving the IV tubing fitment (54, 310) into the mating recess (505) of the fluid delivery device (22, 24, 26, 28) such that the cylindrical body (315) is positioned outside of the mating recess (505) while the substantially hemispherical surface (321) is uniformly mated with the substantially identical surface of the mating recess (505).

14. The method of Claim 13, further comprising:
uniformly mating the substantially hemispherical surface (321) with the substantially identical surface of the mating recess (505) only when the first end (323) of the unitary body (305) is received within a pocket (507) of the mating recess (505) preventing the IV tubing fitment (54, 310) from being misloaded, and
receiving the IV tubing fitment (54, 310) within the pocket (507) of the mating recess (505) such that the first portion (66) of the tubing coupled with the first end (323) of the unitary body (305) is aligned along a center of the mating recess (505).

## Patentansprüche

1. Intravenöse, IV, Schlauchbefestigung (54, 310), umfassend:
einen einheitlichen Körper (305), der so konfiguriert ist, dass er mit einer Flüssigkeitsabgabevorrichtung (22, 24, 26, 28) koppelbar ist, die einen Körper (27), der teilweise eine passende Aussparung (505) bildet, und eine Tür (50, 803) umfasst, wobei die Tür (50, 803) einen Teil der passenden Aussparung (505) bildet, wobei der einheitliche Körper (305) umfasst:
ein erstes Ende (323), das so konfiguriert ist, dass es mit einem ersten Abschnitt (66) eines Rohrs verbindbar ist,
eine im Wesentlichen halbkugelförmige Oberfläche (321), die an das erste Ende (323) angrenzt, und
einen zylindrischen Körper (315), der an die im Wesentlichen halbkugelförmige Oberfläche (321) angrenzt, wobei der zylindrische Körper (315) eine Vielzahl von Flügeln (317) umfasst und so konfiguriert ist, dass er einen zweiten Abschnitt (30) des Rohrs aufnimmt; und
wobei der einheitliche Körper (305) so konfiguriert ist, dass er in der passenden Aussparung (505) der Fluidabgabevorrichtung (22, 24, 26, 28) aufnehmbar ist, so dass:
die im Wesentlichen halbkugelförmige Oberfläche (321) so konfiguriert ist, dass sie gleichmäßig mit einer im Wesentlichen identischen Oberfläche der passenden Ausnehmung (505) zusammenpasst,
der zylindrische Körper (315) so gestaltet ist, dass er außerhalb der passenden Aussparung (505) positioniert ist,
wenn die Tür von einer offenen Position in eine geschlossene Position bewegt wird, während der einheitliche Körper nicht mit der passenden Aussparung in Eingriff steht, die Tür daran gehindert wird, mit dem Körper der Fluidabgabevorrichtung zu koppeln, und
wenn die Tür (50, 803) von der offenen Position in die geschlossene Position bewegt wird, während der einheitliche Körper (305) mit der passenden Aussparung (505) zusammengefügt ist, der einheitliche Körper (305) so konfiguriert ist, dass er in einer Tasche (507) der Fluidabgabevorrichtung (22, 24, 26, 28) eingeschlossen ist.

2. IV-Schlauchbefestigung (54, 310) nach Anspruch 1, wobei der einheitliche Körper (305), wenn er von der passenden Aussparung (505) aufgenommen ist, so konfiguriert ist, dass er sich um eine Längsachse um mindestens 270 Grad im und gegen den Uhrzeigersinn dreht.

3. IV-Schlauchbefestigung (54, 310) nach Anspruch 1 oder Anspruch 2, wobei die im Wesentlichen halbkugelförmige Oberfläche (321) einen Außenrand (319) aufweist, der so konfiguriert ist, dass er mit der Tür (50, 803) der Flüssigkeitsabgabevorrichtung (22, 24, 26, 28) in Eingriff kommt.

4. IV-Schlauchbefestigung (54, 310) nach Anspruch 3, wobei der äußere Rand (319), wenn er mit der Tür (50, 803) der Flüssigkeitsabgabevorrichtung (22, 24, 26, 28) in Eingriff steht, so konfiguriert ist, dass er die Bewegung des einheitlichen Körpers (305) in mindestens einer von einer Längs- und einer Querrichtung beschränkt.

5. IV-Schlauchbefestigung (54, 310) nach einem der Ansprüche 1 bis 4, wobei die im Wesentlichen halbkugelförmige Oberfläche (321) so konfiguriert ist, dass sie nur dann gleichmäßig mit der im Wesentlichen identischen Oberfläche der passenden Aussparung (505) zusammenpasst, wenn das erste Ende (323) des einheitlichen Körpers (305) in der Tasche (507) der passenden Aussparung (505) aufgenommen ist, sodass verhindert wird, dass die IV-Schlauchbefestigung (54, 310) falsch belastet wird.

6. IV-Schlauchbefestigung (54, 310) nach Anspruch 5, wobei, wenn das erste Ende (323) des einheitlichen Körpers (305) in der Tasche (507) der passenden Aussparung (505) aufgenommen ist, der erste Abschnitt (66) des mit dem ersten Ende (323) des einheitlichen Körpers (305) gekoppelten Schlauches entlang einer Mitte der passenden Aussparung (505) ausgerichtet ist.

7. IV-Schlauchbefestigung (54, 310) nach einem der Ansprüche 1 bis 6, wobei die im Wesentlichen halbkugelförmige Oberfläche (321) einen Durchmesser aufweist, der im Wesentlichen gleich einem Durchmesser der Gegenaussparung (505) ist, wobei der Durchmesser der im Wesentlichen halbkugelförmigen Oberfläche (321) so konfiguriert ist, dass er verhindert, dass sich die Flüssigkeitsabgabevorrichtung (22, 24, 26, 28) schließt, wenn die im Wesentlichen halbkugelförmige Oberfläche (321) nicht gleichmäßig mit der im Wesentlichen identischen Oberfläche der Gegenaussparung (505) zusammenpasst.

8. IV-Schlauchbefestigung (54, 310) nach einem der Ansprüche 1 bis 7, wobei die Flüssigkeitsabgabevorrichtung (22, 24, 26, 28) eine Infusionspumpe ist.

9. IV-Schlauchbefestigung (54, 310) nach einem der Ansprüche 1 bis 8, wobei der einheitliche Körper (305) geformt ist.

10. System (20), umfassend:
eine intravenöse, IV, Schlauchbefestigung (54, 310) nach einem der Ansprüche 1 bis 6; und
eine Flüssigkeitsabgabevorrichtung (22, 24, 26, 28), die einen Körper (27) umfasst, der zum Teil eine passende Aussparung (505) bildet, und eine Tür (50, 803), die einen anderen Teil der passenden Aussparung (505) bildet, wobei:
während sich die Tür (50, 803) in einer offenen Position befindet, die Passungsaussparung (505) so konfiguriert ist, dass sie die IV-Schlauchbefestigung (54, 310) aufnimmt, sodass die Passungsaussparung (505) gleichmäßig mit einer im Wesentlichen halbkugelförmigen Oberfläche (321) der IV-Schlauchbefestigung (54, 310) gepaart ist, und ein Abschnitt der IV-Schlauchbefestigung (54, 310) außerhalb des Körpers (27) der Flüssigkeitsabgabevorrichtung (22, 24, 26, 28) positioniert ist;
wenn die Tür in eine geschlossene Position bewegt wird, während die IV-Schlauchbefestigung nicht gleichmäßig mit der passenden Aussparung zusammenpasst, die Tür daran gehindert wird, sich mit dem Körper der Flüssigkeitsabgabevorrichtung zu verbinden; und
wenn die Tür (50, 803) in die geschlossene Position bewegt wird, während das IV-Schlauchbefestigung (54, 310) mit der passenden Aussparung (505) in Eingriff steht, ist die Tür (50, 803) so konfiguriert, dass sie mit dem Körper (27) der Flüssigkeitsabgabevorrichtung (22, 24, 26, 28) gekoppelt ist und die IV-Schlauchbefestigung (54, 310) in einer Tasche (507) einschließt.

11. System nach Anspruch 10, wobei die Tür (50, 803) ein oder mehrere Kopplungselemente (807) enthält, die so konfiguriert sind, dass sie mit einem Abschnitt der IV-Schlauchbefestigung (54, 310) verbunden werden können.

12. System nach Anspruch 10, wobei die Tür (50, 803) so konfiguriert ist, dass sie daran gehindert wird, sich mit dem Körper (27) der Flüssigkeitsabgabevorrichtung (22, 24, 26, 28) zu koppeln, während mindestens ein Abschnitt der IV-Schlauchbefestigung (54, 310) falsch geladen ist.

13. Verfahren zum Laden eines intravenösen (IV) Sets in eine Flüssigkeitsabgabevorrichtung (22, 24, 26, 28), wobei das Verfahren umfasst:
bei einer Flüssigkeitsabgabevorrichtung (22, 24, 26, 28) mit einem Körper (27), der zum Teil eine Eingriffsausnehmung (505) bildet, und einer Tür (50, 803), die einen anderen Teil der Eingriffsausnehmung (505) bildet:
während sich die Tür (50, 803) in einer offenen Position befindet, Aufnahme eines IV-Schlauchbefestigung (54, 310) an der passenden Aussparung (505), so dass eine im Wesentlichen halbkugelförmige Oberfläche (321) des IV-Schlauchbefestigung (54, 310) gleichmäßig mit einer im Wesentlichen identischen Oberfläche der passenden Aussparung (505) zusammenpasst;
während der einheitliche Körper nicht gleichmäßig mit der passenden Ausnehmung in Eingriff gebracht wird, wodurch verhindert wird, dass die Tür mit dem Körper der Flüssigkeitsabgabevorrichtung gekoppelt wird; und
Schließen der Tür (50, 803), sodass die Tür (50, 803) mit dem Körper (27) der Flüssigkeitsabgabevorrichtung (22, 24, 26, 28) gekoppelt wird und eine Umhüllung bildet, die so konfiguriert ist, dass sie mindestens einen Abschnitt der IV-Schlauchbefestigung (54, 310) umgibt,
wobei die IV-Schlauchbefestigung (54, 310) umfasst:
einen einheitlichen Körper (305) mit einem ersten Ende (323), das so konfiguriert ist, dass es mit einem ersten Abschnitt (66) eines Schlauchs verbunden werden kann, wobei die im Wesentlichen halbkugelförmige Oberfläche (321) an das erste Ende (323) angrenzt, und einen zylindrischen Körper (315), der an die im Wesentlichen halbkugelförmige Oberfläche (321) angrenzt, wobei der zylindrische Körper (315) eine Vielzahl von Flügeln (317) aufweist und so konfiguriert ist, dass er einen zweiten Abschnitt (30) des Schlauchs aufnimmt,
wobei das Aufnehmen der IV-Schlauchbefestigung (54, 310) an der passenden Aussparung (505) umfasst:
Aufnehmen der IV-Schlauchbefestigung (54, 310) in der passenden Aussparung (505) der Flüssigkeitsabgabevorrichtung (22, 24, 26, 28), sodass der zylindrische Körper (315) außerhalb der passenden Aussparung (505) positioniert ist, während die im Wesentlichen halbkugelförmige Oberfläche (321) gleichmäßig mit der im Wesentlichen identischen Oberfläche der passenden Aussparung (505) in Eingriff gebracht wird.

14. Verfahren nach Anspruch 13, ferner umfassend:
gleichmäßiges Zusammenpassen der im Wesentlichen halbkugelförmigen Oberfläche (321) mit der im Wesentlichen identischen Oberfläche der passenden Aussparung (505) nur dann, wenn das erste Ende (323) des einheitlichen Körpers (305) in einer Tasche (507) der passenden Aussparung (505) aufgenommen ist, wodurch verhindert wird, dass das IV-Schlauchbefestigung (54, 310) falsch belastet wird, und
Aufnehmen der IV-Schlauchbefestigung (54, 310) in der Tasche (507) der passenden Aussparung (505), so dass der erste Abschnitt (66) des mit dem ersten Ende (323) des einheitlichen Körpers (305) gekoppelten Schlauchs entlang einer Mitte der passenden Aussparung (505) ausgerichtet ist.

## Revendications

1. Raccord de tubulure intraveineuse (54, 310), comprenant :
un corps unitaire (305) configuré pour s'accoupler à un dispositif d'administration de fluide (22, 24, 26, 28) comprenant un corps (27) formant, en partie, une cavité d'accouplement (505) et une porte (50, 803), la porte (50, 803) faisant partie de la cavité d'accouplement (505), le corps unitaire (305) comprenant :
une première extrémité (323) configurée pour s'accoupler à une première partie (66) d'un tuyau,
une surface sensiblement hémisphérique (321) adjacente à la première extrémité (323), et
un corps cylindrique (315) adjacent à la surface sensiblement hémisphérique (321), le corps cylindrique (315) comprenant une pluralité d'ailes (317) et configuré pour recevoir une deuxième partie (30) du tuyau ; et
dans lequel le corps unitaire (305) est configuré pour être reçu dans la cavité d'accouplement (505) du dispositif d'administration de fluide (22, 24, 26, 28) de telle sorte que :
la surface sensiblement hémisphérique (321) est configurée pour s'accoupler uniformément avec une surface sensiblement identique du logement d'accouplement (505),
le corps cylindrique (315) est configuré pour être positionné à l'extérieur de la cavité d'accouplement (505),
lorsque la porte est déplacée d'une position ouverte à une position fermée alors que le corps unitaire n'est pas accouplé avec la cavité d'accouplement, la porte est empêchée de s'accoupler avec le corps du dispositif d'administration de fluide, et
lorsque la porte (50, 803) est déplacée de la position ouverte à la position fermée alors que le corps unitaire (305) est accouplé à la cavité d'accouplement (505), le corps unitaire (305) est configuré pour être enfermé dans une poche (507) du dispositif d'administration de fluide (22, 24, 26, 28).

2. Le raccord de tubulure IV (54, 310) de la revendication 1, dans lequel le corps unitaire (305), lorsqu'il est reçu par la cavité d'accouplement (505), est configuré pour tourner autour d'un axe longitudinal d'au moins 270 degrés dans le sens des aiguilles d'une montre et dans le sens inverse des aiguilles d'une montre.

3. Le raccord de tubulure IV (54, 310) de la revendication 1 ou de la revendication 2, dans lequel la surface sensiblement hémisphérique (321) comprend un bord extérieur (319) qui est configuré pour s'engager dans la porte (50, 803) du dispositif d'administration de fluide (22, 24, 26, 28).

4. Le raccord de tubulure IV (54, 310) de la revendication 3, dans lequel le bord extérieur (319), lorsqu'il est engagé dans la porte (50, 803) du dispositif d'administration de fluide (22, 24, 26, 28), est configuré pour restreindre le mouvement du corps unitaire (305), dans au moins l'une des directions longitudinale et latitudinale.

5. Le raccord de tubulure IV (54, 310) de l'une des revendications 1 à 4, dans lequel la surface sensiblement hémisphérique (321) est configurée pour s'accoupler uniformément avec la surface sensiblement identique de l'évidement d'accouplement (505) uniquement lorsque la première extrémité (323) du corps unitaire (305) est reçue dans la poche (507) de l'évidement d'accouplement (505), empêchant le raccord de tubulure IV (54, 310) d'être mal chargé.

6. Le raccord de tubulure IV (54, 310) de la revendication 5, dans lequel, lorsque la première extrémité (323) du corps unitaire (305) est reçue dans la poche (507) de l'évidement d'accouplement (505), la première portion (66) de la tubulure couplée à la première extrémité (323) du corps unitaire (305) est alignée le long d'un centre de l'évidement d'accouplement (505).

7. Le raccord de tubulure IV (54, 310) de l'une des revendications 1 à 6, dans lequel la surface sensiblement hémisphérique (321) a un diamètre qui est sensiblement égal à un diamètre de la cavité d'accouplement (505), dans lequel le diamètre de la surface sensiblement hémisphérique (321) est configuré pour empêcher le dispositif d'administration de fluide (22, 24, 26, 28) de se fermer lorsque la surface sensiblement hémisphérique (321) n'est pas uniformément accouplée à la surface sensiblement identique de la cavité d'accouplement (505).

8. Le raccord de tubulure IV (54, 310) de l'une des revendications 1 à 7, dans lequel le dispositif d'administration de fluide (22, 24, 26, 28) est une pompe à perfusion.

9. Le raccord de tubulure IV (54, 310) de l'une des revendications 1 à 8, dans lequel le corps unitaire (305) est moulé.

10. Système (20) comprenant
un raccord de tubulure intraveineuse (54, 310) de l'une des revendications 1 à 6 ; et
un dispositif d'administration de fluide (22, 24, 26, 28) comprenant un corps (27) formant, en partie, une cavité d'accouplement (505) et une porte (50, 803) formant une autre partie de la cavité d'accouplement (505), dans lequel :
lorsque la porte (50, 803) est en position ouverte, la cavité d'accouplement (505) est configurée pour recevoir le raccord de tubulure IV (54, 310) de sorte que la cavité d'accouplement (505) est uniformément accouplée à une surface sensiblement hémisphérique (321) de le raccord de tubulure IV (54, 310), et qu'une partie de le raccord de tubulure IV (54, 310) est positionnée à l'extérieur du corps (27) du dispositif d'administration de fluide (22, 24, 26, 28) ;
lorsque la porte est placée en position fermée alors que l'embout de la tubulure IV n'est pas uniformément accouplé avec la cavité d'accouplement, la porte est empêchée de s'accoupler avec le corps du dispositif d'administration de fluide ; et
lorsque la porte (50, 803) est placée en position fermée alors que le raccord de tubulure IV (54, 310) est accouplé à la cavité d'accouplement (505), la porte (50, 803) est configurée pour s'accoupler au corps (27) du dispositif d'administration de fluide (22, 24, 26, 28) en enfermant le raccord de tubulure IV (54, 310) dans une poche (507).

11. Le système de la revendication 10, dans lequel la porte (50, 803) comprend un ou plusieurs éléments de couplage (807) configurés pour se coupler à une partie du raccord de tubulure IV (54, 310).

12. Le système de la revendication 10, dans lequel la porte (50, 803) est configurée pour être empêchée de s'accoupler avec le corps (27) du dispositif d'administration de fluide (22, 24, 26, 28) pendant qu'au moins une partie du raccord de la tubulure IV (54, 310) est mal chargée.

13. Méthode de chargement d'un set intraveineux (IV) dans un dispositif d'administration de fluide (22, 24, 26, 28), la méthode comprenant :
à un dispositif d'administration de fluide (22, 24, 26, 28) comprenant un corps (27) formant, en partie, une cavité d'accouplement (505) et une porte (50, 803) formant une autre partie de la cavité d'accouplement (505) :
lorsque la porte (50, 803) est en position ouverte, recevoir un raccord de tubulure IV (54, 310) au niveau du renfoncement d'accouplement (505) de sorte qu'une surface sensiblement hémisphérique (321) du raccord de tubulure IV (54, 310) soit uniformément accouplée à une surface sensiblement identique du renfoncement d'accouplement (505) ;
lorsque le corps unitaire n'est pas uniformément accouplé avec l'évidement d'accouplement, empêcher la porte de s'accoupler avec le corps du dispositif d'administration de fluide ; et
fermer la porte (50, 803) de sorte que la porte (50, 803) s'accouple avec le corps (27) du dispositif d'administration de fluide (22, 24, 26, 28) et forme une enceinte configurée pour entourer au moins une partie du raccord de tubulure IV (54, 310),
dans lequel le raccord de tubulure IV (54, 310) comprend :
un corps unitaire (305) avec une première extrémité (323) configurée pour se coupler à une première partie (66) d'une tubulure, la surface sensiblement hémisphérique (321) étant adjacente à la première extrémité (323), et un corps cylindrique (315) adjacent à la surface sensiblement hémisphérique (321), le corps cylindrique (315) comprenant une pluralité d'ailes (317) et configuré pour recevoir une deuxième partie (30) de la tubulure,
dans lequel la réception du raccord de tubulure IV (54, 310) au niveau de la cavité d'accouplement (505) comprend :
la réception du raccord de tubulure IV (54, 310) dans le logement d'accouplement (505) du dispositif d'administration de fluide (22, 24, 26, 28) de sorte que le corps cylindrique (315) soit positionné à l'extérieur du logement d'accouplement (505) tandis que la surface sensiblement hémisphérique (321) est uniformément accouplée à la surface sensiblement identique du logement d'accouplement (505).

14. La méthode de la revendication 13, comprenant en outre :
l'accouplement uniforme de la surface sensiblement hémisphérique (321) avec la surface sensiblement identique de la cavité d'accouplement (505) uniquement lorsque la première extrémité (323) du corps unitaire (305) est reçue à l'intérieur d'une poche (507) de la cavité d'accouplement (505) empêchant le raccord de tubulure IV (54, 310) d'être mal chargé, et
recevoir le raccord de tubulure IV (54, 310) dans la poche (507) de la cavité d'accouplement (505) de sorte que la première partie (66) de la tubulure couplée à la première extrémité (323) du corps unitaire (305) soit alignée le long d'un centre de la cavité d'accouplement (505).
